# EUROPEAN PATENT APPLICATION

(11) **EP 4 711 446 A1**
(43) Date of publication of application: **18.03.2026**
(21) Application number: 23940230.8
(22) Date of filing: 26.06.2023
(51) Int. Cl.: C12N 5/071, C12N 5/0735

(54) **CELL CULTURE PRODUCT AND USE THEREOF**

(30) Priority: 05.06.2023 CN 202310654337
(71) Applicant: GUANGXIU-GAOXIN LIFE SCIENCES CO., LTD. HUNAN, Changsha, Hunan 410205 (CN)
(72) Inventor: SUN, Yi, Changsha, Hunan 410205 (CN); LIN, Ge, Changsha, Hunan 410205 (CN); OUYANG, Qi, Changsha, Hunan 410205 (CN); LU, Guangxiu, Changsha, Hunan 410205 (CN)
(74) Representative: Hoffmann Eitle
(86) International application number: PCT/CN2023/102408
(87) International publication number: WO 2024/250349

(57) **Abstract**

Provided are a cell culture product and the use thereof. The cell culture product comprises a hepatic progenitor cell proliferation medium, containing a DMEM/F12 medium, insulin, transferrin, sodium selenite, nicotinamide, 2-phosphate-L-ascorbic acid, a human recombinant albumin, an epidermal cell growth factor, a glycogen synthase kinase 3 inhibitor, a transforming growth factor β receptor inhibitor, lysophosphatidic acid, and sphingosine phosphate.

## Description

### RELATED APPLICATION

This application claims priority to Chinese patent application No. 2023106543373, titled "CELL CULTURE PRODUCT AND USE THEREOF", filed on June 05, 2023, which is incorporated herein by reference in its entirety.

### TECHNICAL FIELD

The present application relates to the field of cell engineering, and in particular, to a cell culture product and use thereof.

### BACKGROUND

The liver is one of the most important organs in the body, performing vital functions such as synthesis, detoxification, excretion, and biotransformation in the body. End-stage liver disease refers to progressive and irreversible liver damage caused by various reasons, which cannot be cured by conventional medical or surgical treatments. The condition is severe, and death is often unavoidable within a short period (6 months to 12 months). Under the influence of various inflammatory factors, most liver functions are extremely exhausted, often accompanied by multiple high-risk complications, with a mortality rate as high as 50% to 80%. According to statistics from the World Health Organization, approximately 2 billion people worldwide have been infected with the hepatitis B virus, among which 350 million are chronic carriers, and about 500,000 to 700,000 people die each year from liver failure, cirrhosis, and primary liver cancer caused by hepatitis B infection. Allogeneic liver transplantation is currently considered the most effective means for treating end-stage liver disease, with a long-term survival rate of 70% to 85% after transplantation. However, the limited supply of liver donors far fails to meet clinical needs. It is reported that the mortality rate of patients awaiting liver transplantation reaches 15% during the waiting period.

Tissue-specific stem/progenitor cells play a very important role in regulating organ formation, regeneration, and tissue homeostasis. Furthermore, these cells have broad application prospects in treating various degenerative diseases and injuries. However, directly isolating most types of adult stem cells from tissues and culturing them *in vitro* remains technically challenging. To achieve cell therapy for liver diseases, it is essential to establish a renewable cell source for hepatocytes. In this context, stem cell-derived hepatocytes show great promise. Embryonic stem cells (ESCs), derived from the inner cell mass of the blastocyst during embryonic development, possess pluripotency and self-renewal capabilities, and have the ability to differentiate into cells of all three germ layers of the body, including hepatocytes. They are "seed cells" in regenerative medicine research. If hepatocytes can be obtained by inducing differentiation from embryonic stem cells, they can provide a broad cell source for the clinical application of hepatocyte therapy.

For example, a conventional technical solution for inducing differentiation of embryonic stem cells into hepatocytes includes as follows.

CN 105385651 B discloses a method for directed induction and differentiation of induced pluripotent stem cells into hepatocytes, including the following steps. Step S1 is providing or preparing induced pluripotent stem cells. Step S2 is a first-stage culture, lasting 3 to 6 days. The induced pluripotent stem cells are initially cultured in RPMI-1640 medium supplemented with human activin A and Wnt3a in the first 1-2 days of the first stage, and the medium is replaced with RPMI-1640 medium supplemented with human activin A and fetal bovine serum in the subsequent culture process of the first stage, to induce differentiation of the induced pluripotent stem cells into definitive endoderm. In the RPMI-1640 medium supplemented with human activin A and Wnt3a, a concentration of human activin A is 100 ng/ml, and a concentration of Wnt3a is 25 ng/ml. In the RPMI-1640 medium supplemented with human activin A and fetal bovine serum, a concentration of human activin A is 10-200 ng/ml, and a volume concentration of fetal bovine serum is 0.2-2%. There are many dead cells in the first few days of the first stage induction. To avoid the impact of dead cells on the induction process, when replacing the medium, the medium should be aspirated as completely as possible and washed once or twice with RPIM-1640 before changing the medium. Step S3 is a second-stage culture, lasting 6-12 days. The cells obtained from step S2 are initially cultured in KO/DMEM medium supplemented with keratinocyte growth factor and fetal bovine serum in the first 1-4 days of the second stage. In the subsequent culture process of the second stage, the medium is replaced with KO/DMEM medium supplemented with serum replacement, L-glutamine, non-essential amino acids, β-mercaptoethanol, and dimethyl sulfoxide, to induce directed differentiation of the cultured cells into hepatic lineage cells. In the KO/DMEM medium supplemented with keratinocyte growth factor and fetal bovine serum, a concentration of keratinocyte growth factor is 15-100 ng/ml, and a volume concentration of fetal bovine serum is 2-3%. In the KO/DMEM medium supplemented with serum replacement, L-glutamine, non-essential amino acids, β-mercaptoethanol, and dimethyl sulfoxide, a volume concentration of serum replacement is 20%, a concentration of L-glutamine is 1-3 mM, a mass concentration of non-essential amino acids is 0.1-15%, a concentration of β-mercaptoethanol is 0.1-0.12 mM, and a volume concentration of dimethyl sulfoxide is 0.2-2%. The number of cells changes significantly during the second-stage culture relative to previous days. At this time, the amount of medium added should be adjusted according to the number of cells to ensure normal cell growth. Step S4 is a third-stage culture, wherein the cells obtained from step S3 are cultured in L-15 medium supplemented with fetal bovine serum, hepatocyte growth factor, oncostatin, dexamethasone, and L-glutamine for 6-10 days to promote maturation of hepatocytes, with the medium replaced every 24 hours. In the L-15 medium supplemented with fetal bovine serum, hepatocyte growth factor, oncostatin, dexamethasone, and L-glutamine, a volume concentration of fetal bovine serum is 10%, a concentration of hepatocyte growth factor is 10-300 ng/ml, a concentration of oncostatin is 5-150 ng/ml, a concentration of dexamethasone is 0.05-1.2 µM, and a concentration of L-glutamine is 2-3 mM. The entire induction process takes 15-28 days. However, the conventional technical solution has a relatively long induction cycle, yield a small number of cells, and have high production costs.

In view of this, the present application is proposed.

### SUMMARY

Based on this, according to various embodiments of the present application, a cell culture product and use thereof are provided. The technical solution is as follows.

In a first aspect of the present application, a cell culture product is provided. The cell culture product includes a hepatic progenitor cell proliferation medium, wherein the hepatic progenitor cell proliferation medium includes DMEM/F12 medium, 1 µg/mL to 8 µg/mL insulin, 5 µg/mL to 15 µg/mL transferrin, 5 ng/mL to 8 ng/mL sodium selenite, 3 mM to 8 mM nicotinamide, 25 µg/mL to 35 µg/mL 2-phosphate-L-ascorbic acid, 45 µg/mL to 55 µg/mL human recombinant albumin, 5 ng/mL to 15 ng/mL epidermal growth factor, 1 µM to 5 µM glycogen synthase kinase 3 inhibitor, 1 µM to 3 µM transforming growth factor β receptor inhibitor, 2 µM to 7 µM lysophosphatidic acid, and 0.2 µM to 0.8 µM sphingosine phosphate.

In some embodiments, the hepatic progenitor cell proliferation medium includes DMEM/F12 medium, 4.5 µg/mL to 5.5 µg/mL insulin, 8 µg/mL to 12 µg/mL transferrin, 6 ng/mL to 7 ng/mL sodium selenite, 4.5 mM to 5.5 mM nicotinamide, 29 µg/mL to 32 µg/mL 2-phosphate-L-ascorbic acid, 48 µg/mL to 52 µg/mL human recombinant albumin, 7.5 ng/mL to 12.5 ng/mL epidermal growth factor, 2.5 µM to 4 µM glycogen synthase kinase 3 inhibitor, 1.5 µM to 2.5 µM transforming growth factor β receptor inhibitor, 4.5 µM to 6 µM lysophosphatidic acid, and 0.45 µM to 0.6 µM sphingosine phosphate.

In some embodiments, the cell culture product further includes an induction-differentiation medium A for inducing differentiation of human pluripotent stem cells into definitive endoderm cells, or further includes an induction-differentiation medium B for inducing differentiation of the definitive endoderm cells into hepatic progenitor cells.

The induction-differentiation medium A includes an induction-differentiation medium A1 and an induction-differentiation medium A2.

The induction-differentiation medium A1 includes RPTM-1640 medium, 5 ng/mL to 300 ng/mL human activin A and 2 µM to 10 µM GSK-3 inhibitor.

The induction-differentiation medium A2 includes RPTM-1640 medium, 10 ng/mL to 200 ng/mL human activin A and 0.2% to 2% (v/v) fetal bovine serum.

In some embodiments, the induction-differentiation medium B includes an induction-differentiation medium B1 and an induction-differentiation medium B2.

The induction-differentiation medium B1 includes KO/DMEM medium, 15 ng/mL to 100 ng/mL keratinocyte growth factor and 2% to 3% (v/v) fetal bovine serum.

The induction-differentiation medium B2 includes KO/DMEM medium, 10% to 30% (v/v) serum replacement, 1 mM to 3 mM L-glutamine, 0.1 wt% to 15 wt% non-essential amino acids, 0.1 mM to 0.12 mM β-mercaptoethanol and 0.2% to 2% (v/v) dimethyl sulfoxide.

In some embodiments, the human pluripotent stem cells are human embryonic stem cells.

In some embodiments, the cell culture product further includes an induction-differentiation medium C for differentiating the induced hepatic progenitor cells into hepatocyte-like cells. The induction-differentiation medium C includes L-15 medium, 5% to 15% (v/v) fetal bovine serum, 10 ng/mL to 300 ng/mL hepatocyte growth factor, 1 ng/mL to 150 ng/mL oncostatin, 0.05 µM to 1.2 µM dexamethasone, 2 mM to 3 mM L-glutamine, and 5 µM to 20 µM transforming growth factor β receptor inhibitor.

In a second aspect of the present application, a method for preparing hepatic progenitor cells or hepatocyte-like cells is provided. The preparation method includes the step of preparing hepatic progenitor cells or hepatocyte-like cells using the cell culture product according to the first aspect.

In some embodiments, the preparation method includes the following steps:
inducing differentiation of human pluripotent stem cells into endoderm cells using the induction-differentiation medium A;
inducing differentiation of the endoderm cells into hepatic progenitor cells using the induction-differentiation medium B;
performing proliferation culture of the hepatic progenitor cells using the hepatic progenitor cell proliferation medium; and
inducing differentiation of the induced hepatic progenitor cells into hepatocyte-like cells using the induction-differentiation medium C.

In some embodiments, the step of performing proliferation culture of the hepatic progenitor cells using the hepatic progenitor cell proliferation medium includes:
initially culturing the hepatic progenitor cells using the hepatic progenitor cell proliferation medium, enzymatically digesting the obtained initial culture to prepare single cells; and
seeding the single cells into fresh hepatic progenitor cell proliferation medium, and culturing the cells for passaging.

In some embodiments, the preparation method includes one or more of the following technical characteristics:
(1) a time period of initial culture is in a range from 0.5 days to 1.5 days;
(2) an enzyme used for enzymatic digestion is TrypLE;
(3) a seeding density of the single cells is in a range from 1×10⁴ cells/cm² to 5×10⁴ cells/cm²; and
(4) the cells are passaged 1 time to 5 times, a culture time for each passage is in a range from 120 h to 168 h, and the medium is replaced every 24 h to 48 h.

Details of one or more embodiments of the present application are set forth in the following description, and other features, objectives, and advantages of the present application will become apparent from the specification and claims.

### BRIEF DESCRIPTION OF THE DRAWINGS

In order to illustrate the technical solutions in the embodiments of the present application or the prior art more clearly, the following briefly introduces the drawings required for describing the embodiments or the prior art. Obviously, the drawings in the following description are only some embodiments of the present application, and for those of ordinary skill in the art, other drawings can be obtained based on these drawings without creative effort.
Fig. 1 shows morphological diagrams of hESCs at different confluence levels during culture in Example 1 of the present application.
Fig. 2 shows flow cytometry detection of pluripotency-related surface markers in hESCs in Example 1 of the present application.
Fig. 3 shows a schematic diagram of detection of totipotency-related gene expression in hESCs in Example 1 of the present application.
Fig. 4 shows morphological changes of human pluripotent stem cells at various stages in Example 1 of the present application.
Fig. 5 shows expression of marker genes of definitive endoderm at the definitive endoderm stage in Example 1 of the present application.
Fig. 6 shows expression of marker genes of hepatic precursor cells at the hepatic precursor stage in Example 1 of the present application.
Fig. 7 shows expression of marker genes of hepatic precursor cells at the hepatoblast stage in Example 1 of the present application.
Fig. 8 shows expression of marker genes of hepatocytes obtained at the terminal stage in Example 1 of the present application.
Fig. 9 shows immunofluorescence staining diagrams of AFP and ALB in Example 1 of the present application.
Fig. 10 shows detection results of Oil Red O staining in Example 1 of the present application.
Fig. 11 shows ICG endocytosis and exocytosis experiments in Example 1 of the present application.
Fig. 12 shows that hESC-derived hepatocytes have the ability to synthesize glycogen in Example 1 of the present application.
Fig. 13 shows that hESC-derived hepatocytes have the ability to uptake low-density lipoprotein in Example 1 of the present application.
Fig. 14 shows a comparison of indicators at the definitive endoderm induction stage between the method of Example 1 of the present application and the conventional method.
Fig. 15 shows a comparison of marker indicators at the hepatic precursor induction stage between the method of Example 1 of the present application and the conventional method.
Fig. 16 shows a comparison of markers of hepatocytes at the induction-maturation stage between the method of Example 1 of the present application and the conventional method.

### DETAILED DESCRIPTION

The technical solutions in the embodiments of the present application will be clearly and completely described below with reference to the drawings in the embodiments of the present application. Obviously, the described embodiments are only a part of the embodiments of the present application, and not all of them. Based on the embodiments in the present application, all other embodiments obtained by a person of ordinary skill in the art without creative effort shall fall within the protection scope of the present application.

Unless otherwise defined, all technical and scientific terms used herein have the same meaning as commonly understood by those skilled in the art to which the present application belongs. The terms used herein in the specification of the present application are for the purpose of describing specific embodiments only and are not intended to limit the present application.

### Terminology

Unless otherwise stated or there is a contradiction, the terms or phrases used herein have the following meanings:
The present application will be described in further detail below with reference to the drawings, embodiments, and examples. It should be understood that the embodiments and examples are merely intended to illustrate the present application and are not intended to limit the scope of the present application. The embodiments and examples are provided for the purpose of making the content disclosed in the present application more thorough and complete. It should also be understood that the present application may be implemented in many different forms and is not limited to the embodiments and examples described herein, and that various changes or modifications may be made by a person skilled in the art without departing from the spirit and scope of the present application, and the obtained equivalents are within the scope of the present application. Furthermore, in the following description, numerous specific details are set forth in order to provide a more thorough understanding of the present application. It should be understood that the present application may be practiced without one or more of the details.

Unless otherwise defined, all technical and scientific terms used herein have the same meaning as commonly understood by those skilled in the art to which the present application belongs. The terms used herein in the specification of the present application are for the purpose of describing embodiments and examples only and are not intended to limit the present application.

### Terminology

Unless otherwise indicated or contradicted, terms or phrases used herein have the following meanings.

The scope of the terms "and/or" and "or/and" as used herein includes any item in two or more associated listed items, and also includes any and all combinations of the two or more associated listed items. The any and all combinations include a combination of any two associated listed items, any more associated listed items, or all associated listed items. It should be noted that when using at least two conjunction pairings selected from "and/or" and "or/and" to connect at least three items, the technical solution of the present application undoubtedly includes the solutions connected by "logical AND" and the solution connected by "logical OR". For example, "A and/or B" includes three parallel schemes: A, B, and "A + B". For another example, a technical solution of "A and/or B and/or C and/or D" includes any item in A, B, C, and D (i.e., a technical solution connected by "logical OR"), and also includes any and all combinations of A, B, C, and D, which means including a combination of any two items or any three items in A, B, C, and D, and also including a four-item combination of A, B, C, and D (i.e., a technical solution connected by "logical AND").

Unless otherwise specified, "a plurality of", "multiple" and the like related in the present application mean greater than 2 or equal to 2 in number. For example, "one or more" means one or two or more.

The "a combination thereof", "any combination thereof", and the like used in the present application include all suitable combinations of any two or more of the listed items.

In the present application, "suitable" in "suitable combination", "suitable mode", "any suitable mode", and the like, is defined by the ability to implement the technical solution of the present application, solve the technical problem of the present application, and achieve the expected technical effect of the present application.

In the present application, "preferably", "better", and "preferred" are only used to describe embodiments or examples with a better effect. It should be understood that these terms do not limit the scope of the present application.

In the present application, "further", "still further", "particularly", and the like are used for descriptive purposes to indicate differences in contents, but should not be construed as limiting the scope of the present application.

In the present application, "optionally" and "optional" refer to something that is not necessary, i.e., any one selected from the two parallel schemes of "present" or "absent". If multiple "optionally" appear in a technical solution, unless otherwise specified and in the absence of contradictions or interdependencies, each "optionally" is independent.

In the present application, the terms "first", "second", "third", "fourth", etc. in "first aspect", "second aspect", "third aspect", "fourth aspect", etc. are used for descriptive purposes only and are not to be construed as indicating or implying a relative importance or quantity, nor as implying any indication of the importance or quantity of the technical features indicated. Moreover, "first", "second", "third", "fourth", etc. are used merely for purposes of non-exhaustive enumeration and description, and should not be construed as a closed limitation of quantity.

In the present application, the technical features described in the open-ended mode include a closed-ended technical solution consisting of the listed features, and also include an open-ended technical solution including the listed features.

In the present application, when a numerical interval (i.e., a numerical range) is related, unless otherwise stated, the distribution of values that are selectable within the numerical range is considered to be continuous and includes both the endpoints of the numerical interval (i.e., the minimum and maximum values) and each value between these two endpoints. Unless otherwise indicated, when a numerical interval only refers to integers within the numerical interval, including both endpoints of the numerical range and each integer between the two endpoints, it is herein equivalent to the direct recitation of each integer. For example, t is an integer selected from 1 to 10, which means that t is any integer selected from the group consisting of 1, 2, 3, 4, 5, 6, 7, 8, 9 and 10. Moreover, when multiple ranges are provided to describe a feature or characteristic, the ranges may be combined. In other words, unless otherwise indicated, all ranges disclosed herein are to be understood to include any and all subranges subsumed therein.

Unless otherwise specified, the temperature parameter in the present application is allowed to be constant temperature treatment or to vary within a certain temperature interval. It should be understood that the constant temperature treatment allows the temperature to fluctuate within the accuracy range controlled by the instrument. It is allowed to fluctuate in a range such as ±5 °C, ±4 °C, ±3 °C, ±2 °C, and ±1 °C.

In the present application, %(w/w) and wt% both refer to a weight percentage, %(v/v) refers to a volume percentage, and %(w/v) refers to a mass-volume percentage.

All documents mentioned in the present application are incorporated by reference, just as each document is cited separately as a reference. The entire content and purpose of the cited references in the present application are incorporated by reference unless they conflict with the objectives and/or technical solutions of the present application. When a cited reference is involved in the present application, definitions of relevant technical features, terms, nouns, phrases, and the like in the cited reference are also incorporated herein. When a cited reference is involved in the present application, the examples, and preferred embodiments of the cited relevant technical features are also incorporated in the present application as references, but only to the extent that they enable the implementation of the present application. It should be understood that when a reference conflicts with the description of the present application, the present application shall prevail or the reference shall be adaptively modified according to the description of the present application.

### First aspect of the present application

The present application provides a cell culture product. The cell culture product includes a hepatic progenitor cell proliferation medium, wherein the hepatic progenitor cell proliferation medium includes DMEM/F12 medium and 1 µg/mL to 8 µg/mL insulin, 5 µg/mL to 15 µg/mL transferrin, 5 ng/mL to 8 ng/mL sodium selenite, 3 mM to 8 mM nicotinamide, 25 µg/mL to 35 µg/mL 2-phosphate-L-ascorbic acid, 45 µg/mL to 55 µg/mL human recombinant albumin, 5 ng/mL to 15 ng/mL epidermal growth factor, 1 µM to 5 µM glycogen synthase kinase 3 inhibitor, 1 µM to 3 µM transforming growth factor β receptor inhibitor, 2 µM to 7 µM lysophosphatidic acid, and 0.2 µM to 0.8 µM sphingosine phosphate.

In the hepatic progenitor cell proliferation medium of the present application, the concentration of insulin is, for example, 1 µg/mL, 2 µg/mL, 3 µg/mL, 4 µg/mL, 5 µg/mL, 6 µg/mL, 7 µg/mL, or 8 µg/mL; the concentration of transferrin is, for example, 5 µg/mL, 6 µg/mL, 7 µg/mL, 8 µg/mL, 9 µg/mL, 10 µg/mL, 11 µg/mL, 12 µg/mL, 13 µg/mL, 14 µg/mL, or 15 µg/mL; the concentration of sodium selenite is, for example, 5 ng/mL, 5.5 ng/mL, 6 ng/mL, 6.5 ng/mL, 7 ng/mL, 7.5 ng/mL, or 8 ng/mL; the concentration of nicotinamide is, for example, 3 mM, 4 mM, 5 mM, 6 mM, 7 mM, or 8 mM; the concentration of 2-phosphate-L-ascorbic acid is, for example, 25 µg/mL, 26 µg/mL, 27 µg/mL, 28 µg/mL, 29 µg/mL, 30 µg/mL, 31 µg/mL, 32 µg/mL, 33 µg/mL, 34 µg/mL, or 35 µg/mL; the concentration of human recombinant albumin is, for example, 45 µg/mL, 46 µg/mL, 47 µg/mL, 48 µg/mL, 49 µg/mL, 50 µg/mL, 51 µg/mL, 52 µg/mL, 53 µg/mL, 54 µg/mL, or 55 µg/mL; the concentration of epidermal growth factor is, for example, 5 ng/mL, 6 ng/mL, 7 ng/mL, 8 ng/mL, 9 ng/mL, 10 ng/mL, 11 ng/mL, 12 ng/mL, 13 ng/mL, 14 ng/mL, or 15 ng/mL; the concentration of glycogen synthase kinase 3 inhibitor is, for example, 1 µM, 2 µM, 3 µM, 4 µM, or 5 µM; the concentration of transforming growth factor β receptor inhibitor is, for example, 1 µM, 1.2 µM, 1.4 µM, 1.6 µM, 1.8 µM, 2 µM, 2.2 µM, 2.4 µM, 2.6 µM, 2.8 µM, or 3 µM; the concentration of lysophosphatidic acid is, for example, 2 µM, 3 µM, 4 µM, 5 µM, 6 µM, or 7 µM; and the concentration of sphingosine phosphate is, for example, 0.2 µM, 0.3 µM, 0.4 µM, 0.5 µM, 0.6 µM, 0.7 µM, or 0.8 µM.

Optionally, the hepatic progenitor cell proliferation medium includes DMEM/F12 medium and 4.5 µg/mL to 6 µg/mL insulin, 8 µg/mL to 12 µg/mL transferrin, 6 ng/mL to 8 ng/mL sodium selenite, 4.5 mM to 6.5 mM nicotinamide, 29 µg/mL to 35 µg/mL 2-phosphate-L-ascorbic acid, 45 µg/mL to 52 µg/mL human recombinant albumin, 7.5 ng/mL to 12.5 ng/mL epidermal growth factor, 2.5 µM to 4 µM glycogen synthase kinase 3 inhibitor, 1.5 µM to 2.5 µM transforming growth factor β receptor inhibitor, 2 µM to 6 µM lysophosphatidic acid, and 0.4 µM to 0.6 µM sphingosine phosphate.

Optionally, the cell culture product further includes an induction-differentiation medium A for inducing differentiation of human pluripotent stem cells into definitive endoderm cells, or further includes an induction-differentiation medium B for inducing differentiation of the definitive endoderm cells into hepatic progenitor cells.

The induction-differentiation medium A includes a medium A1 and a medium A2.

The medium A1 includes RPTM-1640 medium and 5 ng/mL to 300 ng/mL (for example, 5 ng/mL, 10 ng/mL, 20 ng/mL, 30 ng/mL, 40 ng/mL, 50 ng/mL, 60 ng/mL, 70 ng/mL, 80 ng/mL, 90 ng/mL, 100 ng/mL, 150 ng/mL, 200 ng/mL, 250 ng/mL, 300 ng/mL) human activin A and 2 µM to 10 µM (for example, 2 µM, 3 µM, 4 µM, 5 µM, 6 µM, 7 µM, 8 µM, 9 µM, 10 µM) GSK-3 inhibitor.

The medium A2 includes RPTM-1640 medium and 10 ng/mL to 200 ng/mL (for example, 10 ng/mL, 20 ng/mL, 30 ng/mL, 40 ng/mL, 50 ng/mL, 60 ng/mL, 70 ng/mL, 80 ng/mL, 90 ng/mL, 100 ng/mL, 120 ng/mL, 140 ng/mL, 160 ng/mL, 180 ng/mL, 200 ng/mL) human activin A and 0.2% to 2% (for example, 0.2%, 0.4%, 0.8%, 1.0%, 1.2%, 1.4%, 1.6%, 1.8%, 2%) (v/v) fetal bovine serum.

In the present application, the type of GSK-3 inhibitor is not particularly limited, including but being not limited to CHIR99021. CHIR99021 replaces Wnt3a in the conventional induction system and improves induction efficiency. CHIR99021 is much cheaper than Wnt3a, saving production costs. In addition, adding SB431542 at the terminal induction stage can improve the maturity of hepatocytes, increase the expression efficiency of ALB, make the expression level closer to that of mature hepatocytes, and can shorten the time required for the differentiation of hepatic progenitor cells into hepatocyte-like cells.

Optionally, the induction-differentiation medium B includes an induction-differentiation medium B1 and an induction-differentiation medium B2.

The induction-differentiation medium B1 includes KO/DMEM medium and 15 ng/mL to 100 ng/mL (for example, 15 ng/mL, 20 ng/mL, 25 ng/mL, 30 ng/mL, 35 ng/mL, 40 ng/mL, 45 ng/mL, 50 ng/mL, 55 ng/mL, 60 ng/mL, 65 ng/mL, 70 ng/mL, 75 ng/mL, 80 ng/mL, 85 ng/mL, 90 ng/mL, 95 ng/mL, 100 ng/mL) keratinocyte growth factor and 2% to 3% (for example, 2%, 2.2%, 2.4%, 2.6%, 2.8%, 3%) (v/v) fetal bovine serum.

The induction-differentiation medium B2 includes KO/DMEM medium and 10% to 30% (for example, 10%, 12%, 14%, 16%, 18%, 20%, 22%, 24%, 26%, 28%, 30%) (v/v) serum replacement, 1 mM to 3 mM (for example, 1 mM, 1.2 mM, 1.4 mM, 1.6 mM, 2.0 mM, 2.2 mM, 2.4 mM, 2.6 mM, 2.8 mM, 3 mM) L-glutamine, 0.1 wt% to 15 wt% (for example, 0.1 wt%, 1 wt%, 2 wt%, 3 wt%, 4 wt%, 5 wt%, 6 wt%, 7 wt%, 8 wt%, 9 wt%, 10 wt%, 11 wt%, 12 wt%, 13 wt%, 14 wt%, 15 wt%) non-essential amino acids, 0.1 mM to 0.12 mM (for example, 0.1 mM, 0.11 mM, 0.12 mM) β-mercaptoethanol, and 0.2% to 2% (for example, 0.2%, 0.4%, 0.6%, 0.8%, 1%, 1.2%, 1.4%, 1.6%, 1.8%, 2%) (v/v) dimethyl sulfoxide.

Optionally, the human pluripotent stem cells are human embryonic stem cells. Human embryonic stem cells (hESCs) are isolated from the inner cell mass of the blastocyst during embryonic development. They are pluripotent cells with pluripotency and self-renewal capability and can be indefinitely passaged *in vitro.* In both *in vitro* and *in vivo* environments, human embryonic stem cells have the potential to be induced to differentiate into almost all cell types of the body, including hepatocytes. They are "seed cells" in regenerative medicine research. Hepatocytes derived from human embryonic stem cells simulate the formation process of hepatic lineage cells during differentiation. Hepatocytes derived from human embryonic stem cells are good materials for studying liver development and hepatocyte research. Furthermore, hepatocytes obtained by induced culture from human embryonic stem cells also have broad application prospects in drug screening and drug metabolism research.

Optionally, the cell culture product further includes an induction-differentiation medium C for differenting the induced hepatic progenitor cells into hepatocyte-like cells, wherein the induction-differentiation medium C includes L-15 medium and 5% to 15% (for example, 5%, 6%, 7%, 8%, 9%, 10%, 11%, 12%, 13%, 14%, 15%) (v/v) fetal bovine serum, 10 ng/mL to 300 ng/mL (for example, 10 ng/mL, 20 ng/mL, 30 ng/mL, 40 ng/mL, 50 ng/mL, 60 ng/mL, 70 ng/mL, 80 ng/mL, 90 ng/mL, 100 ng/mL, 150 ng/mL, 200 ng/mL, 250 ng/mL, 300 ng/mL) hepatocyte growth factor, 1 ng/mL to 150 ng/mL (for example, 1 ng/mL, 5 ng/mL, 10 ng/mL, 20 ng/mL, 30 ng/mL, 40 ng/mL, 50 ng/mL, 60 ng/mL, 70 ng/mL, 80 ng/mL, 90 ng/mL, 100 ng/mL, 110 ng/mL, 120 ng/mL, 130 ng/mL, 140 ng/mL, 150 ng/mL) oncostatin, 0.05 µM to 1.2 µM (for example, 0.05 µM, 0.1 µM, 0.2 µM, 0.3 µM, 0.4 µM, 0.5 µM, 0.6 µM, 0.7 µM, 0.8 µM, 0.9 µM, 1.0 µM, 1.1 µM, 1.2 µM) dexamethasone, 2 mM to 3 mM (for example, 2 mM, 2.1 mM, 2.2 mM, 2.3 mM, 2.4 mM, 2.5 mM, 2.6 mM, 2.7 mM, 2.8 mM, 2.9 mM, 3 mM) L-glutamine, and 5 µM to 20 µM (for example, 5 µM, 6 µM, 7 µM, 8 µM, 9 µM, 10 µM, 11 µM, 12 µM, 13 µM, 14 µM, 15 µM, 16 µM, 17 µM, 18 µM, 19 µM, 20 µM) transforming growth factor β receptor inhibitor.

### Second aspect of the present application

The present application provides a method for preparing hepatic progenitor cells or hepatocyte-like cells. The preparation method includes the step of preparing hepatic progenitor cells or hepatocyte-like cells using the cell culture product according to the first aspect.

Optionally, the preparation method includes the following steps:
inducing differentiation of human pluripotent stem cells into endoderm cells using the induction-differentiation medium A;
inducing differentiation of the endoderm cells into hepatic progenitor cells using the induction-differentiation medium B;
performing proliferation culture of the hepatic progenitor cells using the hepatic progenitor cell proliferation medium; and
inducing differentiation of the induced hepatic progenitor cells into hepatocyte-like cells using the induction-differentiation medium C.

In the present application, a multi-stage directed induction-differentiation technology is adopted, in which some factors are replaced with small molecules. This technology further improves the efficiency of induction-differentiation, shortens the time of the maturation stage, and greatly increases the yield of terminal hepatocytes induced through passaging by adding a hepatic progenitor proliferation stage, thereby saving production costs, significantly increasing cell yield, and reducing the requirement for initial seed cells. Furthermore, cells at the hepatic progenitor cell stage can be cryopreserved, and subsequent induction can be performed when needed, increasing the flexibility of induction time and shortening cell preparation time. Moreover, the initial seed cells, i.e., human pluripotent stem cells, are proliferated using a feeder-free, animal-derived component-free culture system, and hepatic progenitor cells and hepatocytes can be obtained simply by adding the induction media at the corresponding stages. The obtained hepatocytes have stable performance and, after testing, have high purity, without the problem of feeder cell contamination.

Optionally, the step of performing proliferation culture of the hepatic progenitor cells using the hepatic progenitor cell proliferation medium includes:
initially culturing the hepatic progenitor cells using the hepatic progenitor cell proliferation medium, enzymatically digesting the obtained initial culture to prepare single cells; and
seeding the single cells into fresh hepatic progenitor cell proliferation medium, and culturing the cells for passaging.

Optionally, the preparation method has one or more of the following technical characteristics:
(1) a time period of initial culture is in a range from 0.5 days to 1.5 days (for example, 0.5 days, 0.6 days, 0.7 days, 0.8 days, 0.9 days, 1.0 day, 1.1 days, 1.2 days, 1.3 days, 1.4 days, 1.5 days);
(2) an enzyme used for enzymatic digestion is TrypLE;
(3) a seeding density of the single cells is in a range from 1×10⁴ cells/cm² to 5×10⁴ cells/cm²; and
(4) the cells are passaged 1 time to 5 times (for example, 1 passage, 2 passages, 3 passages, 4 passages, 5 passages), a culture time for each passage is in a range from 120 h to 168 h (for example, 120 h, 125 h, 130 h, 135 h, 140 h, 145 h, 150 h, 160 h, 165 h, 168 h), and the medium is replaced every 24 h to 48 h (for example, 24 h, 26 h, 28 h, 30 h, 32 h, 34 h, 36 h, 38 h, 40 h, 42 h, 44 h, 46 h, 48 h).

The embodiments of the present application will be described in detail below with reference to the examples. It should be understood that these examples are only for illustrative purposes and not intended to limit the scope of the present application. The experimental procedures without conditions noted in the following examples are preferably conducted according to the guidelines given in the present application, and may be performed according to the experimental manual or routine conditions in the art, according to the conditions suggested by the manufacturer, or according to the experimental procedures known in the art.

In the examples described below, the measurement parameters related to the components of the raw materials may be slightly deviated from the weighing accuracy range, unless otherwise specified. When temperature and time parameters are related to, acceptable deviation caused by the instrument test accuracy or operational accuracy is allowed.

The following are specific examples.

### Example 1

### I. Reagents and equipment used

hESCs (human embryonic stem cells): clinical-grade 90 series human embryonic stem cells established by the National Engineering Research Center for Human Stem Cells were selected.

### Cell culture reagents:

Human activin A (R&D, 338-AC-050, i.e., R&D Systems company, product number 338-AC-050, similarly hereinafter), Wnt3a (R&D, 1324-WN-010), fetal bovine serum (Gibico, 10099-141), keratinocyte growth factor (KGF) (R&D, 251-KG-050), insulin-transferrin-sodium selenite (ITS) (Sigma, I1884), nicotinamide (Sigma, L7260), 2-phosphate-L-ascorbic acid (Sigma, 49752), human recombinant albumin (Sigma, A9731), epidermal growth factor (EGF) (Sigma, 236-GMP), glycogen synthase kinase 3 inhibitor (CHIR99021) (Sigma, SML1046), transforming growth factor β receptor inhibitor (SB431542) (Sigma, S4317), lysophosphatidic acid (LPA) (Sigma, L7260) and sphingosine phosphate (S1P) (Sigma, 73914), TrypLE (Gibco, A1285901), serum replacement (Life, N10828-028), L-glutamine (Gibco, 25030-081), non-essential amino acids (Gibco, 1140-050), β-mercaptoethanol (Gibco, 21385-023), dimethyl sulfoxide (DMSO) (Sigma, D2660), hepatocyte growth factor (HGF) (R&D, 294-HG-025), oncostatin (OSM) (R&D, 295-OM-050), dexamethasone (Calbiochem, 265005), RPTM-1640 (Life, 11875-085), KO/DMEM (Gibco, A1286101), L-15 (Gibco, 21083-027), LN-521 (BioLamina, LN521), NutriStem hESC xeno-free (XF) medium (Biological Industries, 05-100-1A).

### II. Specific Induction and Identification Process

### (I) Culture of human pluripotent stem cells

After thawing, the human pluripotent stem cell line was inoculated into culture flasks pre-coated with LN521 matrix. The NutriStem hESC xeno-free (XF) medium was replaced daily, and the cells were passaged every three days. After passaging three times, when the human pluripotent stem cells were in the best growth state, the medium was replaced with induction medium.

The human pluripotent stem cells detected to express pluripotency-related genes could be used for the next experiment. The specific detection was as follows.

### 1. Detection of pluripotency-related genes

Undifferentiated hESCs were collected, RNA thereof was extracted for detection of totipotency-related genes or differentiation genes. The method was as follows:

### 1) RNA extraction

The corresponding cells were collected, and 1 mL of TRIZOL was added to lyse the cells. 0.2 mL of chloroform was added per 1 mL of TRIZOL. The mixture was incubated at room temperature for 2-3 min, and then was subjected to high-speed cryogenic centrifugation at 12,000 × g at 4°C for 15 min. After centrifugation, the mixture was separated into three layers, with RNA present in the aqueous layer. The aqueous layer was transferred to a clean tube. 0.5 mL of isopropanol was added and mixed thoroughly. The mixed sample was incubated at room temperature for 10 min and subjected to high-speed cryogenic centrifugation at 12,000 g or below at 4°C for 10 min. The upper suspension was removed. The RNA precipitate was washed once with 75% v/v% ethanol (i.e., the volume fraction of ethanol is 75%, similarly hereinafter), and subjected to high-speed cryogenic centrifugation at 7,500 g or below at 2°C to 8°C for 5 min. The RNA precipitate was briefly dried (i.e., air-dried for 5-10 min). It was dissolved in RNase-free DEPC water. After complete dissolution, the RNA concentration was measured with a UV spectrophotometer.

### 2) Reverse transcription (RT) to synthesize cDNA

A RevertAid^{™} first strand cDNA synthesis kit (Fermentas Life Sciences) was used according to the instructions thereof. Specific steps were as follows: 1 µg total RNA and 1 µL oligo (dT) primer were added in an EP tube, with RNase-free water added to a final reaction volume of 12 µL. The reaction mixture was denatured at 70°C for 5 min, and quickly cooled on ice. Then, 4 µL of 5× reverse transcription reaction buffer, 2 µL of 10 mM dNTP, and 1 µL of RNase inhibitor were added. The resultant was mixed gently, centrifuged rapidly, and incubated at 25°C for 5 min. 1 µL of AMV reverse transcriptase was added. The final reaction volume was 20 µL. The resultant was reacted at 25°C for 10 min, 42°C for 60 min, and 70°C for 10 min, and cooled to 4°C. The obtained cDNA was stored at -20°C or used for PCR amplification of target genes.

Using RNA as a template, PCR amplification was performed with commonly used β-actin primers to confirm no gDNA contamination.

Then cDNA was used as a template for RT-PCR to detect the expression of pluripotency-related genes OCT3/4, NANOG, and SOX2, definitive endoderm marker genes SOX17 and FoxA2, hepatic precursor stage and hepatic progenitor stage genes AFP, HNF4α and CK7, hepatocyte maturation stage marker genes ALB and AAT, or representative genes of differentiation of three germ layers, with β-actin as the positive control for the reaction system. PCR reaction conditions were: 95°C for 1 min and 30 sec; 30 cycles (94°C for 40 sec, 54°C-64°C for 40 sec, and 72°C for 40 sec); extension at 72°C for 7 min.

### (II) Large-scale directed induction-differentiation culture of human embryonic stem cell-derived liver cells

1. Thawing of human pluripotent stem cells: Cells were taken from the liquid nitrogen tank, immediately thawed in a constant temperature water bath at 37°C, centrifuged to discard supernatant. NutriStem hESC xeno-free (XF) medium was added to resuspend the cell pellet, cells were inoculated into culture flasks pre-coated with LN521 matrix, the medium was replaced daily, and cells were passaged every three days.
2. Culture of human pluripotent stem cells: After confirming no mycoplasma or bacterial contamination, cells were digested with Tryple digestion enzyme and centrifuged, the supernatant was removed, cells were resuspended with NutriStem hESC xeno-free (XF) medium and inoculated into culture flasks pre-coated with LN521 matrix. The cells were passaged, during which the medium was replaced every 24 hours, and cell growth status was observed daily.
3. Induction-differentiation of human pluripotent stem cells: After the cells were passaged three times, when the human pluripotent stem cells were in the best growth state, the medium was replaced with induction medium. The specific induction process was as follows.

### First stage (lasting 3 days): induction of human pluripotent stem cells into definitive endoderm

The induction medium for day 1 (induction-differentiation medium A1) was basal medium RPTM-1640 supplemented with human activin A and glycogen synthase kinase 3 inhibitor (CHIR99021), wherein the usage concentration of human activin A was 100 ng/mL and the usage concentration of glycogen synthase kinase 3 inhibitor (CHIR99021) was 6 µM.

The induction medium for day 2 (induction-differentiation medium A2) was basal medium RPTM-1640 supplemented with human activin A and fetal bovine serum, wherein the final concentration of human activin A was 100 ng/mL, and the final concentration of fetal bovine serum was 2% (v/v).

The induction medium for day 3 (induction-differentiation medium A2) was basal medium RPTM-1640 supplemented with human activin A and fetal bovine serum, wherein the final concentration of human activin A was 100 ng/mL, and the final concentration of fetal bovine serum was 2% (v/v).

There were many dead cells in the first few days of the first stage induction. To avoid the impact of dead cells on the induction process, when replacing the medium, the medium should be aspirated as completely as possible and washed once or twice with RPIM-1640 before changing the medium.

### Second stage (lasting 7 days): directed differentiation into hepatic lineage

The medium used for the first 2 days of the second stage (induction-differentiation medium B1) was basal medium KO/DMEM supplemented with 50 ng/mL keratinocyte growth factor and a final concentration of 2% (v/v) fetal bovine serum. The number of cells changed significantly during the second-stage culture process compared to previous days. At this time, the amount of medium added should be adjusted according to the cell number to ensure normal cell growth.

The medium for the subsequent 5 days (induction-differentiation medium B2) was basal medium KO/DMEM supplemented with serum replacement (SR), L-glutamine, non-essential amino acids, β-mercaptoethanol, and dimethyl sulfoxide. The medium was replaced every 24 hours, wherein the final concentration of serum replacement is 20% (v/v), the final concentration of L-glutamine is 1 mM, the final concentration of non-essential amino acids is 1% (v/v), the final concentration of β-mercaptoethanol is 0.1 mM, and the final concentration of dimethyl sulfoxide is 1% (v/v).

### Third stage (lasting 5 days): hepatic progenitor cell proliferation stage (using hepatic progenitor cell proliferation medium)

In the third stage, cells were first cultured with basal medium DMEM/F12 supplemented with insulin-transferrin-sodium selenite (ITS), nicotinamide, 2-phosphate-L-ascorbic acid, human recombinant albumin, epidermal growth factor (EGF), glycogen synthase kinase 3 inhibitor (CHIR99021), transforming growth factor β receptor inhibitor (SB431542), lysophosphatidic acid (LPA), sphingosine phosphate (S1P) for 1 day, then digested into single cells with TrypLE, and the single cells were passaged at a density of 1×10⁴ cells/cm² to 5×10⁴ cells/cm². The cells were cultured for 4 days, and the medium was replaced every 24 hours during the culture process. At this stage, cells could also be digested using Tryple digestion enzyme and passaged for 1-5 times. After expanding the cells to a certain number, the obtained hepatic progenitor cells could be cryopreserved with CS10 for backup. When cells were urgently needed, the hepatic progenitor cells could be thawed, and only maturation induction was required to obtain hepatocytes. This could greatly shorten the induction time. At this stage, hepatocyte maturation induction could also be performed directly without cryopreservation.

Wherein, the final concentration of insulin was 6 µg/mL, the final concentration of transferrin was 12 µg/mL, the final concentration of sodium selenite was 8 ng/mL, the final concentration of nicotinamide was 6 mM, the final concentration of 2-phosphate-L-ascorbic acid was 35 µg/mL, the final concentration of human recombinant albumin was 45 µg/mL, the final concentration of epidermal growth factor (EGF) was 12 ng/mL, the final concentration of glycogen synthase kinase 3 inhibitor (CHIR99021) was 3 µM, the final concentration of transforming growth factor β receptor inhibitor (SB431542) was 2.5 µM, the final concentration of lysophosphatidic acid (LPA) was 2 µM, and the final concentration of sphingosine phosphate (S1P) was 0.4 µM.

### Fourth stage (lasting 5 days): promotion of hepatocyte maturation stage

The medium (induction-differentiation medium C) was basal medium L-15 supplemented with fetal bovine serum, hepatocyte growth factor, oncostatin, dexamethasone, L-glutamine, and transforming growth factor β receptor inhibitor. The medium was changed every 24 hours. Wherein, the final concentration of fetal bovine serum was 10%, the final concentration of hepatocyte growth factor was 10 ng/mL, the final concentration of oncostatin was 20 ng/mL, the final concentration of dexamethasone was 0.05 µM, the final concentration of L-glutamine was 2 mM, and the concentration of transforming growth factor β receptor inhibitor was 12 µM.

The preparation method of the present application achieves efficient expansion of cells at the hepatic progenitor stage. First, it increases the yield of hepatocyte-like cells obtained per batch at the terminal stage. Second, it reduces the requirement for the amount of initial seed cells for induction. Third, the expanded hepatic progenitor stage cells can be cryopreserved to establish a hepatic progenitor cell bank. During production, hepatic progenitor cells can be directly thawed, expanded by passaging, and then directly induced to differentiate into hepatocyte-like cells, requiring only subsequent 5-7 days of maturation induction, which greatly shortens the hepatocyte preparation cycle and makes hepatocyte preparation more efficient. On the other hand, replacing some cytokines with small molecules greatly reduces the production cost of cell preparation and improves the quality of hepatocytes.

### (III) Identification of hepatocytes

### 1. Observation of differentiation during cell induction process

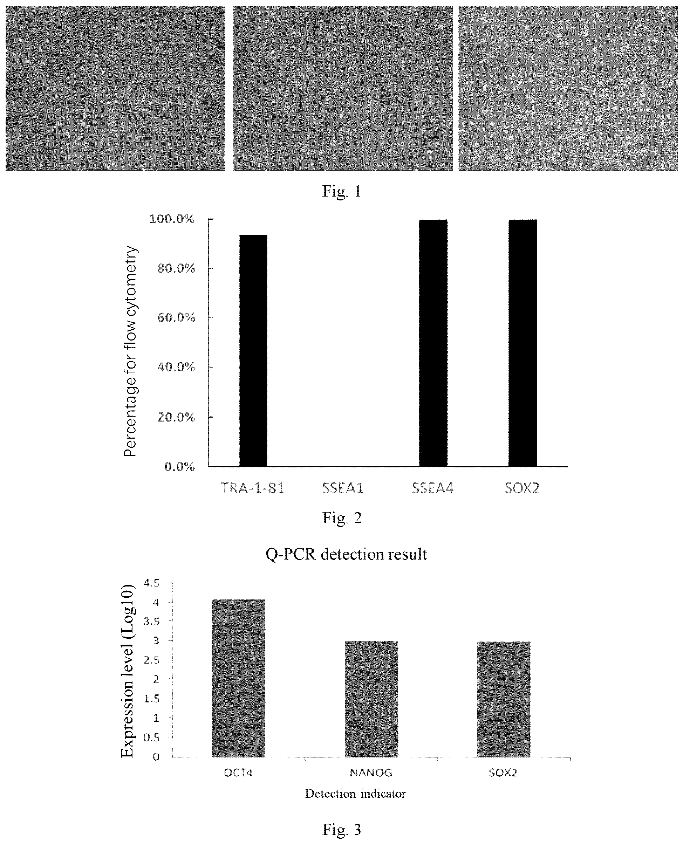

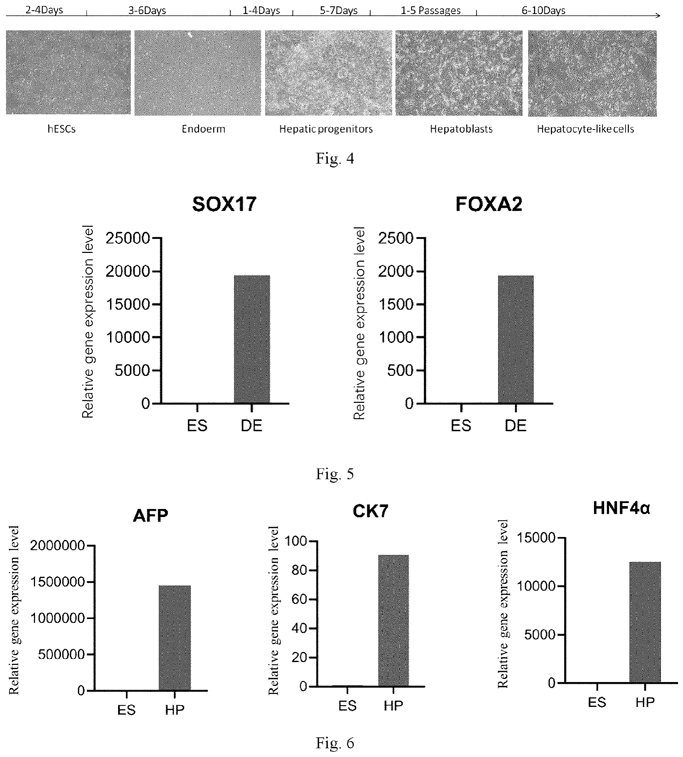

Starting from the thawing of human pluripotent stem cells, the cell growth status, adherence, and differentiation status were observed daily under a microscope. hESCs had a high nuclear-to-cytoplasmic ratio and were tightly arranged. Fig. 1 shows the morphology of hESCs at different confluence levels. After induction began, cells began to undergo morphological changes. After 3 days of induction, cells entered the definitive endoderm stage, becoming flattened and oval. After entering the second stage induction, cells began to enter the hepatocyte specification stage, gradually becoming polygonal with clear boundaries. The third stage was the hepatic progenitor cell stage, where cells were polygonal, small in volume, and grew faster. The fourth stage was the hepatocyte maturation stage, where cell colonies became more dense, had increased cell volume, being cuboidal, with abundant cytoplasm, having one or more nuclei, and obvious nucleoli. The morphological changes of cells at each stage are shown in Fig. 4, which indicated that the cultured cells were more similar to parenchymal hepatic cells.

### 2. Flow cytometry detection

Direct-labeled flow cytometry was used to detect the expression of pluripotency-related proteins in hESCs. Cells were collected, and then supernatant was discarded. 2 mL of 1% FBS buffer was added. Cells were gently pipetted to resuspend, centrifuged at 300g for 5 min, and the supernatant was removed. TRA-1-81 (BD, 560884), SSEA1 (BD, 560127), SSEA4 (BD, 560126) flow antibodies and corresponding isotype control antibodies (BD, 555584, 555578, 553474) were diluted with 1% FBS buffer to a volume of 100 µL for each antibody. The antibodies were added to the test sample tubes, one tube added the detection antibody, another added the isotype control. The antibodies were mixed thoroughly with cells, incubated at 2°C to 8°C protected from light for 30 minutes. 1 mL of 1% FBS buffer was added to wash cells and centrifuged at 300g for 5 minutes, and then the supernatant was removed, which was conducted twice. 300 µL of 1% FBS buffer was added to resuspend cells in each test sample tube which was protected from light, and filtered through a membrane. The corresponding fluorescence channel was selected according to the fluorescence information labeled on the flow antibodies. The experimental results were analyzed using a BD Accuri C6 instrument. For SOX2 detection, after collecting cells, 0.2 mL of Fixation/Permeabilization working solution (BD, cat. no. 51-2090KZ-125mL) was added, mixed thoroughly with cells, fixed/permeabilized at 4°C protected from light for 20 minutes, and centrifuged at 300g for 5 min, and the supernatant was discarded. 1 mL of Perm/Wash Buffer working solution (BD, cat. no. 554723-100mL) was directly added to wash cells and centrifuged at 300g for 5 min, and the supernatant was discarded, which was conducted twice. Afterward, SOX2 (BD, 561593) flow antibody and corresponding isotype control antibody (BD, 557721) were added. Fig. 2 shows flow cytometry detection results of pluripotency-related surface markers in hESCs. The results indicated that hESCs specifically highly expressed human pluripotency-related proteins TRA-1-81, SSEA4, SOX2, but did not express SSEA1, meeting the identification criteria for hESCs.

### 3. RT-QPCR

hESCs before initial induction and cells on the last day of each induction stage were collected for detection. Cells were lysed with TRIzol to extract RNA. Reverse transcription was performed using a reverse transcription kit according to the instructions. Each sample contained 1 µg of RNA. The PCR reaction system included 0.2 µL of cDNA, 10 µL of PCR Green Mix, 7.8 µL water, 1 µL of 1 µM forward primer and 1 µL of 1 µM reverse primer. The sequences of the verification primer pairs were shown in SEQ ID NOs. 1-2, SEQ ID NOs.3-4, SEQ ID NOs.5-6, SEQ ID NOs.7-8, SEQ ID NOs.9-10, SEQ ID NOs.11-12, SEQ ID NOs.13-14, SEQ ID NOs.15-16, SEQ ID NOs.17-18, and SEQ ID NOs.19-20, respectively. The procedure was as follows: 95°C for 5 min; then 45 cycles of 95°C for 10 s, 58°C for 10 s, 72°C for 10 s. The results are shown in Fig. 3, Fig. 5, Fig. 6, Fig. 7, and Fig. 8. Fig. 5 to Fig. 8 show the detection results of specific gene expression at different induction stages compared to hESCs. ES represented pluripotent stem cells (expressing OCT4 gene, NANOG gene, and SOX2 gene), DE represented definitive endoderm cells (expressing FoxA2 gene and SOX17 gene), HP represented hepatic progenitor cells (expressing AFP gene, HNF4α gene and CK7 gene), HPLC represented hepatocyte-like cells (expressing ALB gene and AAT gene).
OCT4 forward primer (SEQ ID No.1): agcgaaccag tatcgagaac
OCT4 reverse primer (SEQ ID No.2): ttacagaacc acactcggac
NANOG forward primer (SEQ ID No.3): ggtctcgtatttgctgcatcg
NANOG reverse primer (SEQ ID No.4): acactcggtgaaatcagggt
SOX2 forward primer (SEQ ID No.5): agctacagca tgatgcagga
SOX2 reverse primer (SEQ ID No.6): ggtcatggag ttgtactgca
SOX17 forward primer (SEQ ID No.7): ggaccgcacggaatttgaac
SOX17 forward primer (SEQ ID No.8): ggacaccaccgaggaaatgg
FOXA2 forward primer (SEQ ID No.9): ggaacaccac tacgccttca ac
FOXA2 reverse primer (SEQ ID No. 10): tcatgttgct cacggaggag ta
AFP forward primer (SEQ ID No. 11): ccatgtacat gagcactgtt g
AFP reverse primer (SEQ ID No. 12): ctccaataac tcctggtatc c
HNF4α forward primer (SEQ ID No. 13): catggccaag attgacaacc t
HNF4α reverse primer (SEQ ID No.14): ttcccatatg ttcctgcatc ag
CK-7 forward primer (SEQ ID No. 15): catggccaag attgacaacc t
CK-7 reverse primer (SEQ ID No. 16): ttcccatatg ttcctgcatc ag
ALB forward primer (SEQ ID No.17): gatgagatgc ctgctgactt gc
ALB reverse primer (SEQ ID No. 18): cacgacagag taatcaggat gcc
AAT forward primer (SEQ ID No.19): gatgagatgc ctgctgactt gc
AAT reverse primer (SEQ ID No.20): cacgacagag taatcaggat gcc
TTR forward primer (SEQ ID No.21): cgtgcatgtgttcagaaaggctg
TTR reverse primer (SEQ ID No.22): ctcctcagttgtgagcccatgc
RBP4 forward primer (SEQ ID No.23): agtgagcagcttccgagtcaag
RBP4 reverse primer (SEQ ID No.24): gagaactccgcgacgatgttgt

### 4. Immunofluorescence detection of AFP and ALB expression

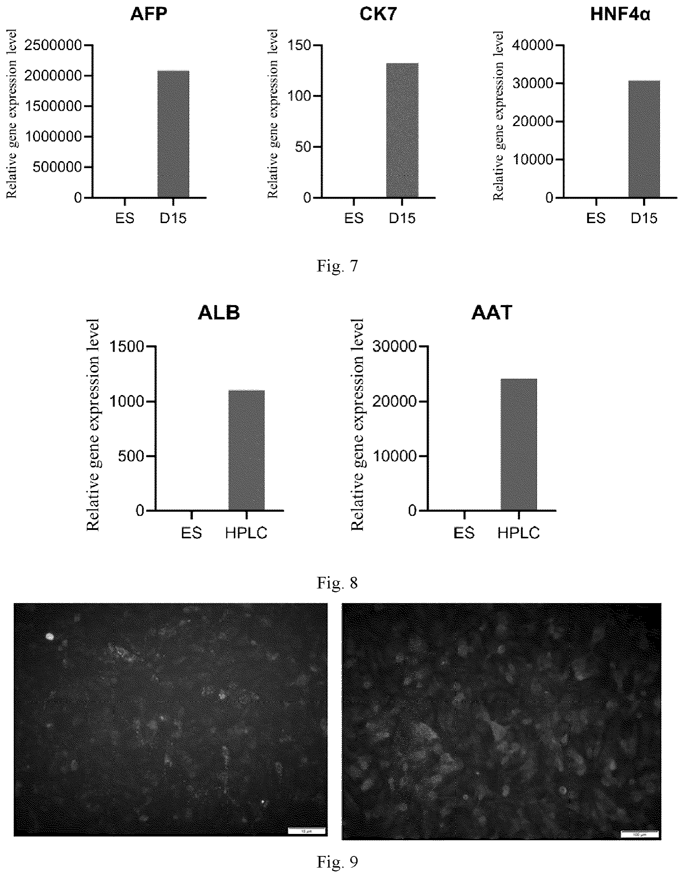

Cells were fixed with 4 wt% (in a mass concentration, similarly hereinafter) paraformaldehyde at room temperature for 15 minutes, then permeabilized with 0.2 v/v% Triton X100 for 15 min, blocked with 4 v/v% normal goat serum for 1 hour. Primary antibody was diluted in blocking solution and the cells were incubated with the primary antibody at 4°C overnight. Then, the cells were incubated with secondary antibody at room temperature for one hour. DAPI was added for counterstaining for 5 minutes. Positive cells were observed under a fluorescence microscope. The results are shown in Fig. 9. Fig. 9 shows that the hepatocyte-like cells induced by the present method expressed liver-specific markers ALB and AFP.

### 5. Detection of ICG uptake and release by human pluripotent stem cell-derived hepatocytes

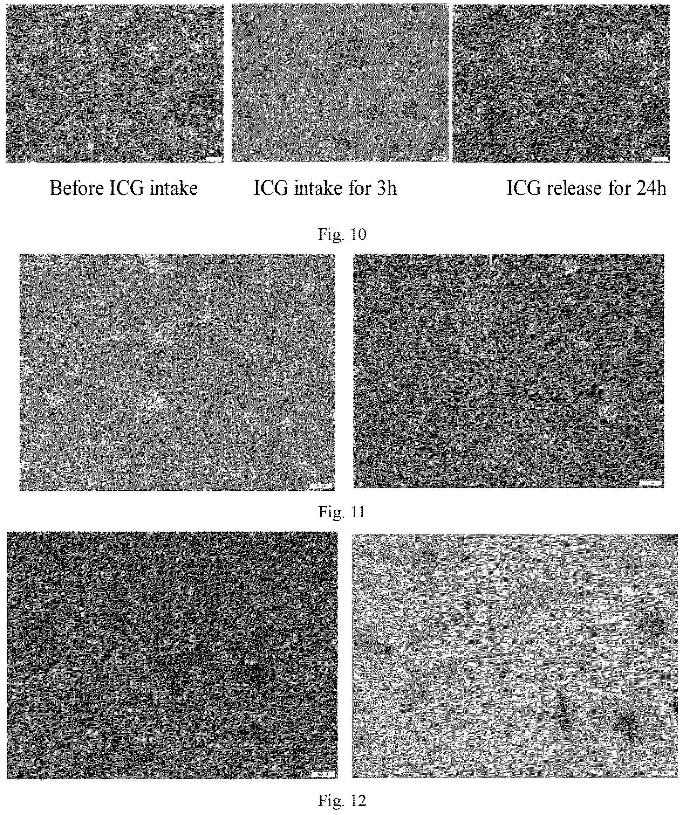

ICG was dissolved in dimethyl sulfoxide at a concentration of 5 mg/mL, then diluted with induction-differentiation medium C to a final concentration of 1 mg/mL. Human pluripotent stem cell-derived hepatocytes were cultured in differentiation medium containing ICG routinely for 90 min, and washed with phosphate buffer. The ICG uptake by cells was observed and recorded under a microscope. Then, the medium was replaced with ordinary medium, and cells were cultured for more than 6 hours, and ICG release was observed. The results are shown in Fig. 10. Fig. 10 shows the microscopic observation after incubating with ICG dye for 90 min, showing that the cells clearly contained ICG dye. After removing the ICG dye for 4 h to 6 h, the cells returned colorless. These results indicated that human pluripotent stem cell-derived hepatocytes had the ability to uptake and secrete ICG-like dyes.

### 6. Oil Red O staining experiment to detect the ability of hepatocytes to synthesize triglycerides

Human pluripotent stem cell-derived hepatocytes were treated with 4 wt% paraformaldehyde. 5% Oil Red stock solution was mixed with distilled water at a volume ratio of 3:2, the mixed solution was filtered, and then added to the cell culture dish. The hepatocytes were stained under protection from light for 15-20 min. After removing Oil Red dye, the sample was rinsed with 60% isopropanol solution for a few seconds, then washed with PBS, and observed under a microscope and photographed. The results are shown in Fig. 11. Fig. 11 shows the microscopic observation of orange-red lipid droplets in the cells after Oil Red O staining, indicating that human pluripotent stem cell-derived hepatocytes had the ability to synthesize and store lipids.

### 7. Periodic acid-Schiff reaction to detect glycogen synthesis

Human pluripotent stem cell-derived hepatocytes were treated with 4 wt% paraformaldehyde and 1 v/v% Triton X100, then stained according to the instructions of the Periodic acid-Schiff staining system (Sigma-Aldrich, Pittsburgh, USA). The results are shown in Fig. 12. Fig. 12 shows that most cells were stained purple-red after glycogen staining by the PAS method, indicating that human pluripotent stem cell-derived hepatocytes had strong glycogen synthesis and storage capacity.

### 8. Low-density lipoprotein uptake experiment

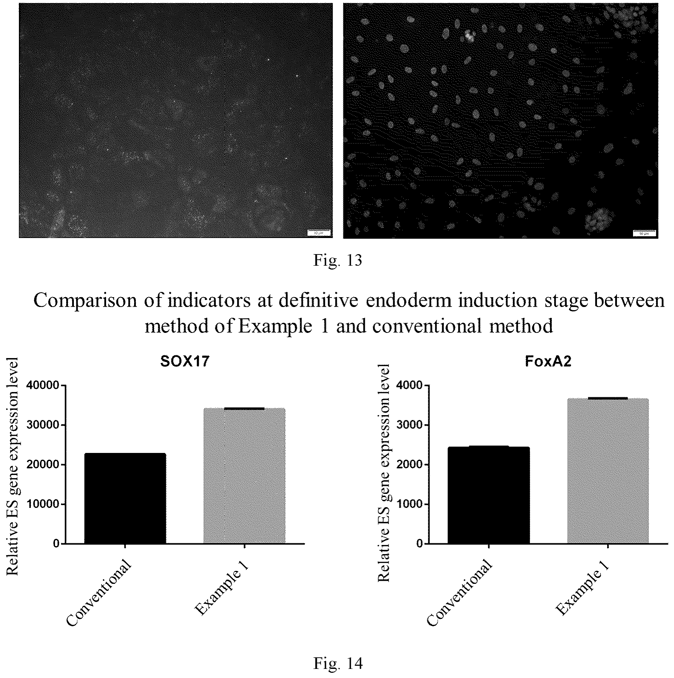

Cells were cultured in serum-free medium containing 10 µg/mL Alexa-Flour 488-labeled LDL for 3 to 4 hours and counterstained with DAPI. Fluorescence was observed under a fluorescence microscope. The results are shown in Fig. 13. Fig. 13 shows fluorescence microscopy results after incubating cells with LDL, showing that most cells contained green fluorescence-labeled LDL particles, indicating that human pluripotent stem cell-derived hepatocytes had lipid uptake function.

### 9. Experimental comparison of CHIR99021 with Wnt3a

In the present application, a control group for the above induction process was also set up. In the control group, during the induction culture process, Wnt3a was used instead of CHIR99021 in induction medium A1 (the control group was recited as "conventional" in Fig. 14), and rest components were the same as "3. Induction-differentiation of human pluripotent stem cells". The results are shown in Fig. 14. Fig. 14 shows the impact of using CHIR99021 and Wnt3a respectively on inducing differentiation of hepatocytes on day 3 (induction into definitive endoderm). In Fig. 14, each group of bar graphs, from left to right, includes "ES", "conventional" and "Example 1", wherein "Example 1" refers to the operation according to "II. Specific Induction and Identification Process" above.

### 10. Experimental comparison of adding SB431542 at the terminal induction stage

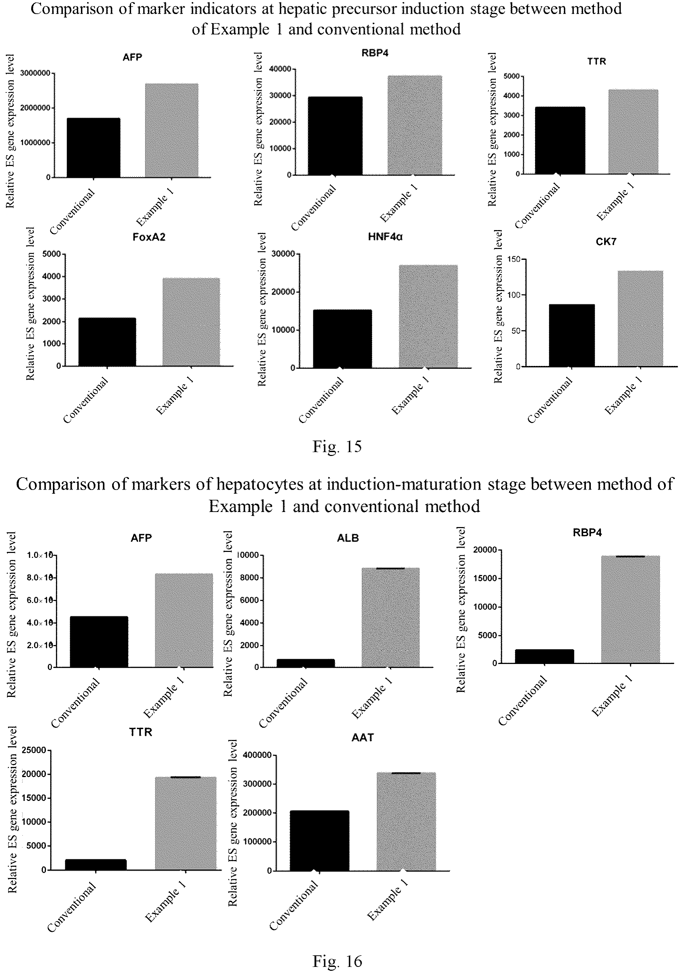

In the present application, SB431542 was further omitted from the induction-differentiation medium C of the control group (the control group was recited as "conventional" in Figs. 15 and 16) based on the above control group, and the rest components were the same as "3. Induction-differentiation of human pluripotent stem cells". The hepatocyte indicators at the terminal stage were compared in Fig. 15. Adding the small molecule SB431542 at the terminal stage greatly improved the induction efficiency and maturity of hepatocytes. In Figs. 15 and 16, each group of bar graphs, from left to right, includes "ES", "conventional" and "Example 1", wherein "Example 1" refers to the operation according to "II. Specific Induction and Identification Process " above.

The present application provides an optimized method for directed induction and differentiation of human pluripotent stem cells into hepatic progenitor cells and hepatocytes by rationally using cell growth factors. During the operation process, changes of morphology and number of cells can be observed at any time to ensure normal induction, and the required hepatic progenitor cells and hepatocytes can be induced quickly and efficiently. This large-scale preparation method for human pluripotent stem cell-derived hepatic progenitor cells and hepatocytes adopts a feeder-free culture system. Hepatic progenitor cells and hepatocytes can be obtained simply by adding the induction media at the corresponding stages, and after testing, the cells have high purity. By using the above large-scale preparation method for human pluripotent stem cell-derived hepatic progenitor cells and hepatocytes, cells at the hepatic progenitor stage undergo extensive self-proliferation. After obtaining hepatic progenitor cells, they can be cryopreserved. When hepatocytes are urgently needed, they can be thawed and inducd to promote maturation. The subsequent induction has short cycle and is efficient, and the obtained hepatocytes have stable performance without the problem of stromal cell contamination.

The technical features of the above-described embodiments can be combined arbitrarily. For the sake of brevity, not all possible combinations of the technical features in the above embodiments are described. However, as long as there is no contradiction in the combination of these technical features, they should be considered as within the scope of this description.

The above-described embodiments only represent several implementation modes of the present application, and the description thereof is specific and detailed, but should not be construed as limiting the scope of the patent. It should be pointed out that for those of ordinary skill in the art, several modifications and improvements can be made without departing from the concept of the present application, and these all fall within the protection scope of the present application. Therefore, the protection scope of the patent of the present application shall be subject to the appended claims.

## Claims

1. A cell culture product, wherein the cell culture product comprises a hepatic progenitor cell proliferation medium, wherein the hepatic progenitor cell proliferation medium comprises DMEM/F12 medium, about 1 µg/mL to 8 µg/mL insulin, about 5 µg/mL to 15 µg/mL transferrin, about 5 ng/mL to 8 ng/mL sodium selenite, about 3 mM to 8 mM nicotinamide, about 25 µg/mL to 35 µg/mL 2-phosphate-L-ascorbic acid, about 45 µg/mL to 55 µg/mL human recombinant albumin, about 5 ng/mL to 15 ng/mL epidermal growth factor, about 1 µM to 5 µM glycogen synthase kinase 3 inhibitor, about 1 µM to 3 µM transforming growth factor β receptor inhibitor, about 2 µM to 7 µM lysophosphatidic acid, and about 0.2 µM to 0.8 µM sphingosine phosphate.

2. The cell culture product according to claim 1, wherein the hepatic progenitor cell proliferation medium comprises DMEM/F12 medium, about 4.5 µg/mL to 6 µg/mL insulin, about 8 µg/mL to 12 µg/mL transferrin, about 6 ng/mL to 8 ng/mL sodium selenite, about 4.5 mM to 6.5 mM nicotinamide, about 29 µg/mL to 35 µg/mL 2-phosphate-L-ascorbic acid, about 45 µg/mL to 52 µg/mL human recombinant albumin, about 7.5 ng/mL to 12.5 ng/mL epidermal growth factor, about 2.5 µM to 4 µM glycogen synthase kinase 3 inhibitor, about 1.5 µM to 2.5 µM transforming growth factor β receptor inhibitor, about 2 µM to 6 µM lysophosphatidic acid, and about 0.4 µM to 0.6 µM sphingosine phosphate.

3. The cell culture product according to claim 1 or 2, wherein the cell culture product further comprises an induction-differentiation medium A for inducing differentiation of human pluripotent stem cells into definitive endoderm cells, or further comprises an induction-differentiation medium B for inducing differentiation of the definitive endoderm cells into hepatic progenitor cells;
wherein the induction-differentiation medium A comprises an induction-differentiation medium A1 and an induction-differentiation medium A2;
the induction-differentiation medium A1 comprises RPTM-1640 medium, about 5 ng/mL to 300 ng/mL human activin A and about 2 µM to 10 µM GSK-3 inhibitor;
the induction-differentiation medium A2 comprises RPTM-1640 medium, about 10 ng/mL to 200 ng/mL human activin A and about 0.2% to 2% (v/v) fetal bovine serum.

4. The cell culture product according to claim 3, wherein the induction-differentiation medium B comprises an induction-differentiation medium B1 and an induction-differentiation medium B2;
the induction-differentiation medium B1 comprises KO/DMEM medium, about 15 ng/mL to 100 ng/mL keratinocyte growth factor and about 2% to 3% (v/v) fetal bovine serum;
the induction-differentiation medium B2 comprises KO/DMEM medium, about 10% to 30% (v/v) serum replacement, about 1 mM to 3 mM L-glutamine, about 0.1 wt% to 15 wt% non-essential amino acids, about 0.1 mM to 0.12 mM β-mercaptoethanol and about 0.2% to 2% (v/v) dimethyl sulfoxide.

5. The cell culture product according to claim 3, wherein the human pluripotent stem cells are human embryonic stem cells.

6. The cell culture product according to any one of claims 1 to 5, wherein the cell culture product further comprises an induction-differentiation medium C for differentiating the induced hepatic progenitor cells into hepatocyte-like cells, wherein the induction-differentiation medium C comprises L-15 medium, about 5% to 15% (v/v) fetal bovine serum, about 10 ng/mL to 300 ng/mL hepatocyte growth factor, about 1 ng/mL to 150 ng/mL oncostatin, about 0.05 µM to 1.2 µM dexamethasone, about 2 mM to 3 mM L-glutamine, and about 5 µM to 20 µM transforming growth factor β receptor inhibitor.

7. A method for preparing hepatic progenitor cells or hepatocyte-like cells, wherein the method comprises the step of preparing hepatic progenitor cells or hepatocyte-like cells using the cell culture product according to any one of claims 1 to 6.

8. The method according to claim 7, wherein the method comprises the following steps:
inducing differentiation of human pluripotent stem cells into endoderm cells using the induction-differentiation medium A;
inducing differentiation of the endoderm cells into hepatic progenitor cells using the induction-differentiation medium B;
performing proliferation culture of the hepatic progenitor cells using the hepatic progenitor cell proliferation medium; and
inducing differentiation of the induced hepatic progenitor cells into hepatocyte-like cells using the induction-differentiation medium C.

9. The method according to claim 8, wherein the step of performing proliferation culture of the hepatic progenitor cells using the hepatic progenitor cell proliferation medium comprises:
initially culturing the hepatic progenitor cells using the hepatic progenitor cell proliferation medium, enzymatically digesting the obtained initial culture to prepare single cells; and
seeding the single cells into fresh hepatic progenitor cell proliferation medium, and culturing the cells for passaging.

10. The method according to claim 9, wherein the method has one or more of the following technical characteristics:
(1) a time period of initial culture is in a range from about 0.5 days to 1.5 days;
(2) an enzyme used for enzymatic digestion is TrypLE;
(3) a seeding density of the single cells is in a range from about 1×10⁴ cells/cm² to 5×10⁴ cells/cm²; and
(4) the cells are passaged about 1 time to 5 times, a culture time for each passage is in a range from about 120 h to 168 h, and the medium is replaced every about 24 h to 48 h.
